# EUROPEAN PATENT APPLICATION

(11) **EP 1 400 243 A1**
(43) Date of publication of application: **24.03.2004**
(21) Application number: 03255860.3
(22) Date of filing: 18.09.2003
(51) Int. Cl.: A61K 31/415, A61K 31/33, A61P 29/00

(54) **Calcium-activated K channel activator**

(30) Priority: 19.09.2002 JP 2002272662; 14.03.2003 JP 2003070298; 24.07.2003 JP 2003278699
(71) Applicant: TANABE SEIYAKU CO., LTD., Chuo-ku, Osaka-shi, Osaka 541-8505 (JP)
(72) Inventor: Kono, Rikako, Towa City Coop, Saitama-shi Saitama 330-0854 (JP); Kohnomi, Shuntarou, Toda-shi Saitama 335-0015 (JP); Aihara, Hajime, Saitama-shi Saitama 337-0051 (JP); Hosaka, Toshihiro, Tokyo 110-0015 (JP); Kashiwagi, Toshihiko, Toda-shi Saitama 335-0015 (JP)
(74) Representative: Cresswell, Thomas Anthony

(57) **Abstract**

There is disclosed the use of a compound of formula (I): 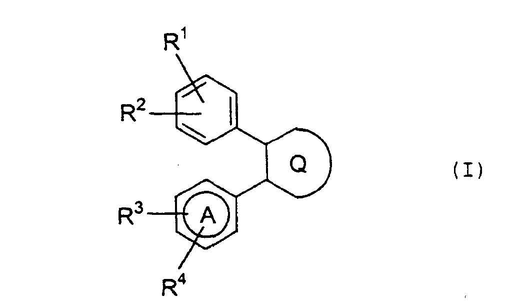 or a pharmaceutically acceptable salt thereof, for the manufacture of a medicament for the prophylaxis or treatment of a disorder or disease responsive to opening of the large conductance calcium-activated K channel.

## Description

### BACKGROUND OF THE INVENTION

### 1. FIELD OF THE INVENTION

This invention relates to an excellent large conductance calcium-activated K channel opener, which is useful for treatment of disorders or diseases such as pollakiuria, urinary incontinence, cerebral infarction, subarachnoid hemorrhage, and the like.

### 2. BACKGROUND ART

Potassium is the most abundant intracelluar cation, and is very important in maintaining physiological homeostasis. Potassium channels are present in almost all vertebrate cells, and the potassium influx through these channels is indispensable for maintaining hyperpolarized resting membrane potential.

Large conductance calcium activated potassium channels (also BK channels or maxi-K channels) are expressed especially in neurons and smooth muscle cells. Because both of the increase of intracellular calcium concentration and membrane depolarization can activate maxi-K channels, maxi-K channels have been thought to play a pivotal role in regulating voltage-dependent calcium influx. Increase in the intracellular calcium concentration mediates many processes such as release of neurotransmitters, contraction of smooth muscles, cell growth and death, and the like. Actually, the opening of maxi-K channels causes strong membrane hyperpolarization, and inhibits these calcium-induced responses thereby. Accordingly, by inhibiting various depolarization-mediated physiological responses, a substance having an activity of opening maxi-K channels is expected to have potential for the treatment of diseases such as cerebral infarction, subarachnoid hemorrhage, pollakiuria, urinary incontinence, and the like.

Also, there has been disclosed that a medicine which opens a BK channel can be an agent for treatment of sexual function disorder such as erectile dysfunction, etc. as disclosed in International Publication WO00/34244.

There have been various reports on a large conductance calcium-activated potassium channel opener. For example, pyrrole derivatives have been disclosed in International Publications WO96/40634, a furan derivative has been disclosed in Japanese Provisional Patent Publication No. 2000-351773, a nitrogen-containing 5-membered derivative in which the nitrogen is substituted by phenyl or benzyl has been disclosed in International Publication WO98/04135 and a diphenyltriazole derivative has been disclosed in Journal of Medicinal Chemistry, vol. 45, pp.2942-2952, 2002.

Also, cyclooxygenase-2 inhibitors such as celecoxib, valdecoxib, etc. have been used as a medicine for inflammatory diseases such as rheumatoid arthritis as disclosed in Japanese PCT provisional publications No. 9-506350 and No. 9-500372. However, there is no report of these compounds for use as a large conductance calcium-activated. K channel opener.

### SUMMARY OF THE INVENTION

An object of the present invention is to provide an excellent large conductance calcium-activated K channel opener, which is useful for treatment of disorders or diseases such as pollakiuria, urinary incontinence, cerebral infarction, subarachnoid hemorrhage, and the like.

The present inventors have studied intensively to solve the problems, and as a result, they have found that a certain kind of compounds which have been known as a cyclooxygenase-2 inhibitor have an excellent large conductance calcium-activated K channel opening activity, whereby they have accomplished the present invention.

That is, the present invention is as mentioned below:
(1) A large conductance calcium-activated K channel opener comprising a compound of the formula (I): 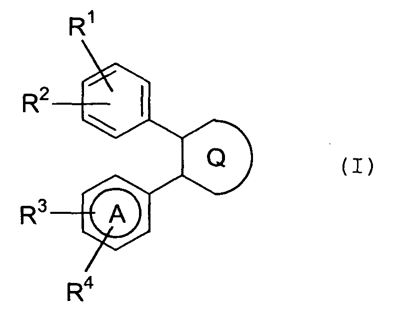 wherein R¹ is a halogen, aminosulfonyl, an alkylsulfonyl or an alkanoylaminosulfonyl; R² is hydrogen or a halogen; R³ and R⁴ may be the same or different from each other and each is hydrogen, a halogen, an alkyl or an alkoxy; Ring A is benzene, pyridine or a cycloalkane, and Ring Q is 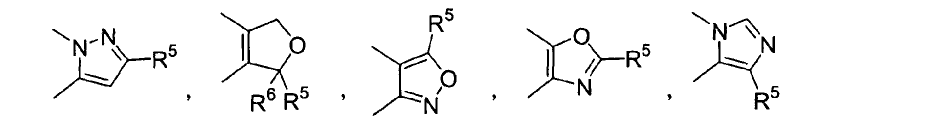 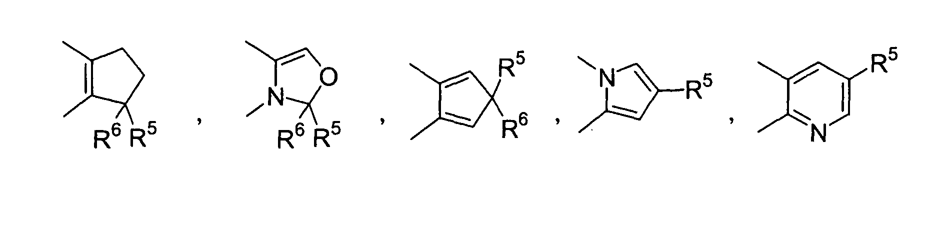 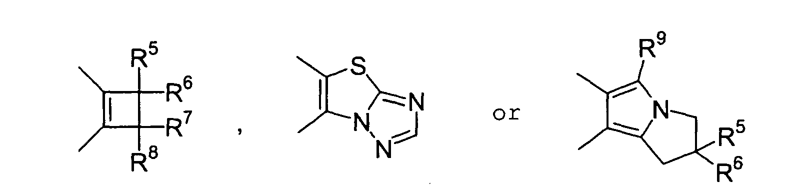 where R⁵ is a halogen, an alkyl or a haloalkyl; R⁶ is hydrogen or an alkyl; or R⁵ and R⁶ may be combined to each other to form oxo; R⁷ and R⁸ are hydrogen or may be combined to each other to form oxo; and R⁹ is a carboxyalkyl,
   or Ring Q and Ring A may be combined to each other to form a fused ring of the formula: 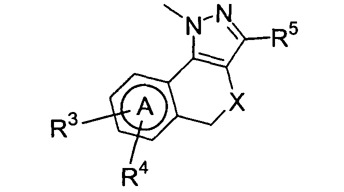 where X is sulfur or oxygen, and R³, R⁴ and R⁵ have
   the same meanings as defined above,
   or a pharmaceutically acceptable salt thereof as an active ingredient.
(2) The large conductance calcium-activated K channel opener as mentioned in the above (1), wherein the opener contains a compound of the formula (II): 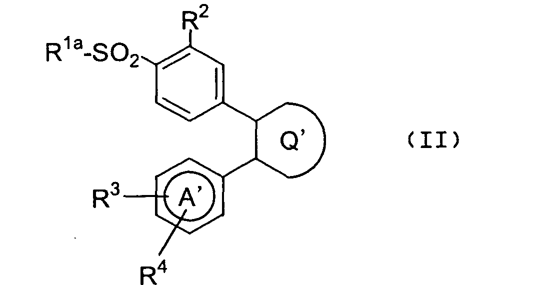 wherein R^{1a} is amino, an alkyl or an alkanoylamino; R² is hydrogen or a halogen; R³ and R⁴ may be the same or different from each other and each is hydrogen, a halogen, an alkyl or an alkoxy; Ring A' is benzene or a cycloalkane, and Ring Q' is 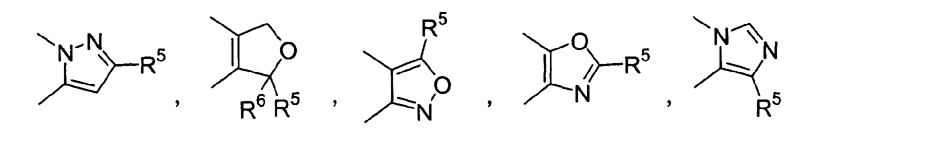 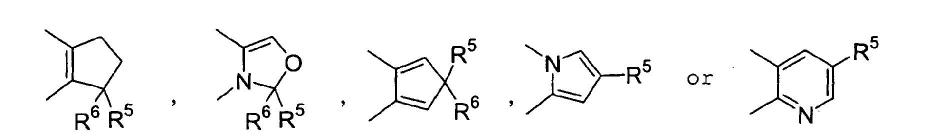 where R⁵ is a halogen, an alkyl or a haloalkyl; R⁶ is hydrogen or an alkyl; or R⁵ and R⁶ may be combined to each other to form oxo,
   or a pharmaceutically acceptable salt thereof as an active ingredient.
(3) The large conductance calcium-activated K channel opener as mentioned in the above (1), wherein the opener contains at least one compound selected from the group consisting of the compounds (1) to (46) mentioned below, or a pharmaceutically acceptable salt thereof as an active ingredient.
(4) The large conductance calcium-activated K channel opener as mentioned in the above (1), wherein the opener contains at least one compound selected from the group consisting of the compounds (1) to (3), (10), (21) to (23), (36), (37) and (43) to (45) mentioned below, or a pharmaceutically acceptable salt thereof as an active ingredient.
(5) A large conductance calcium-activated K cannel opener according to any one of the above-mentioned (1) to (4), wherein the opener is an agent for the prophylaxis or treatment of pollakiuria or urinary incontinence.

### DESCRIPTION OF THE PREFERRED EMBODIMENTS

The active ingredient compounds of the present invention have common structural characteristic feature in which they have 4- to 6-membered ring to which two 6-membered rings are substituted at the adjacent portions thereof. According to this structural characteristic feature, these compounds have an excellent large conductance calcium-activated K channel opening activity.

In the following, respective groups represented by the respective symbols in the specification are explained.

The halogen of R¹, R², R³ and R⁵ means fluorine, chlorine, bromine and iodine, and fluorine and chlorine are preferred.

The alkylsulfonyl of R¹ is exemplified by an alkylsulfonyl having 1 to 4 carbon atoms, specifically methylsulfonyl, ethylsulfonyl, etc., and preferably methylsulfonyl.

The alkanoylaminosulfonyl of R¹ is exemplified by an alkanoylaminosulfonyl having 2 to 4 carbon atoms, specifically acetylaminosulfonyl, propionylaminosulfonyl, etc. The alkanoylamino portion may form a salt with an alkali metal (such as sodium).

The alkyl of R³, R⁴, R⁵, R⁶ and R^{1a} is exemplified by an alkyl having 1 to 4 carbon atoms, specifically methyl, ethyl, etc., and preferably methyl.

The alkoxy of R³ and R⁴ is exemplified by an alkoxy having 1 to 4 carbon atoms, specifically methoxy, ethoxy, etc., and preferably methoxy.

The cycloalkane of Ring A and Ring A' is exemplified by a cycloalkane having 3 to 7 carbon atoms, specifically cyclopentane, cyclohexane, etc., and preferably cyclohexane.

The haloalkyl of R⁵ is exemplified by a haloalkyl having 1 to 4 carbon atoms, specifically chloromethyl, trifluoromethyl, etc.

The carboxyalkyl of R⁹ is exemplified by a carboxyalkyl having 1 to 4 carbon atoms, specifically carboxymethyl.

The alkanoylamino of R^{1a} is exemplified by an alkanoylamino having 2 to 4 carbon atoms, specifically acetylamino, propionylamino, etc. Moreover, the alkanoylamino portion may form a salt with an alkali metal (such as sodium).

Examples of pharmaceutically acceptable salts of the compound as an active ingredient may include a salt with an inorganic base such as sodium salt, potassium salt, etc., and a salt with an inorganic acid such as hydrochloride, sulfate, etc.

The compounds (1) to (18) which are active ingredients of the present invention have the chemical structure as shown in the following Table 1.

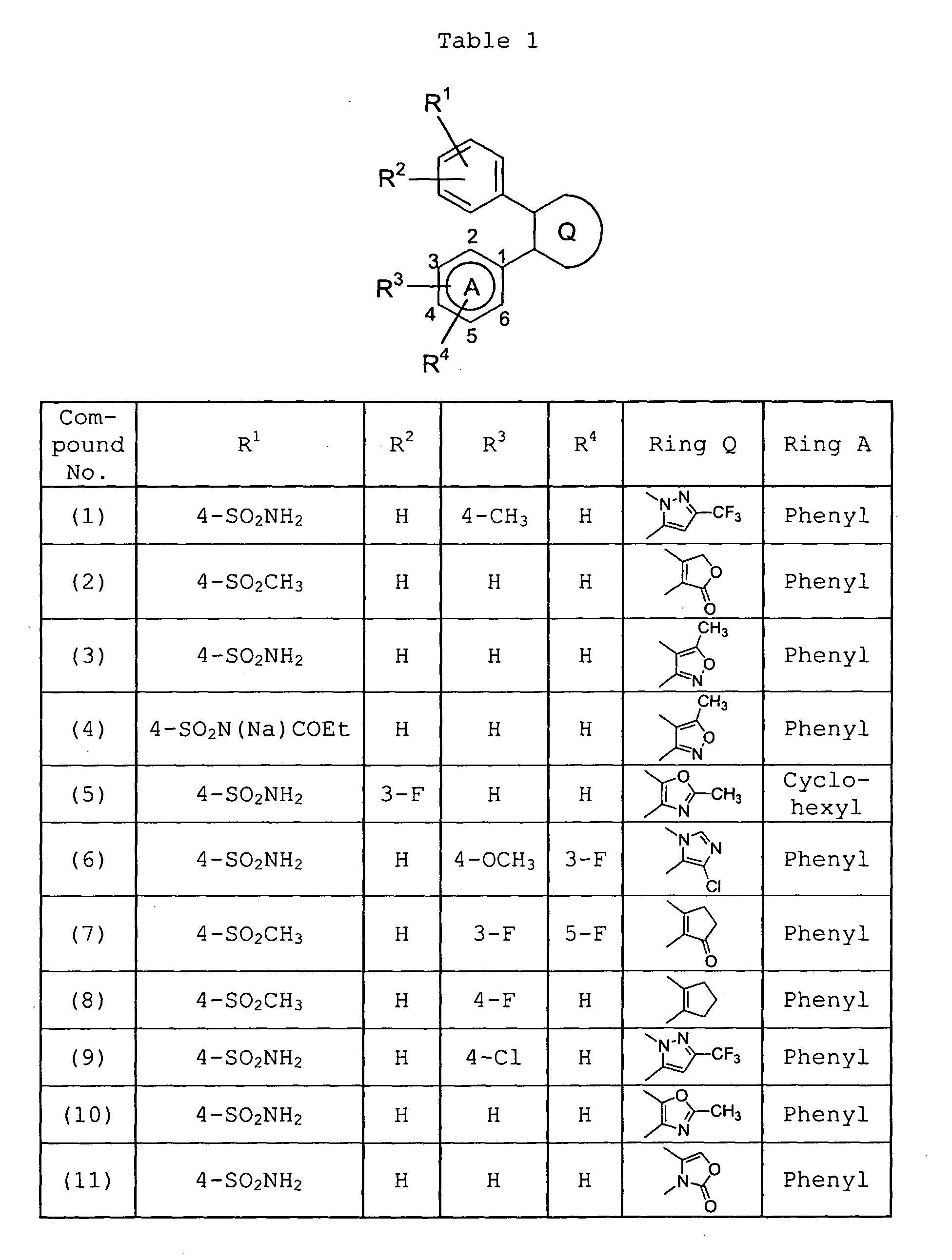

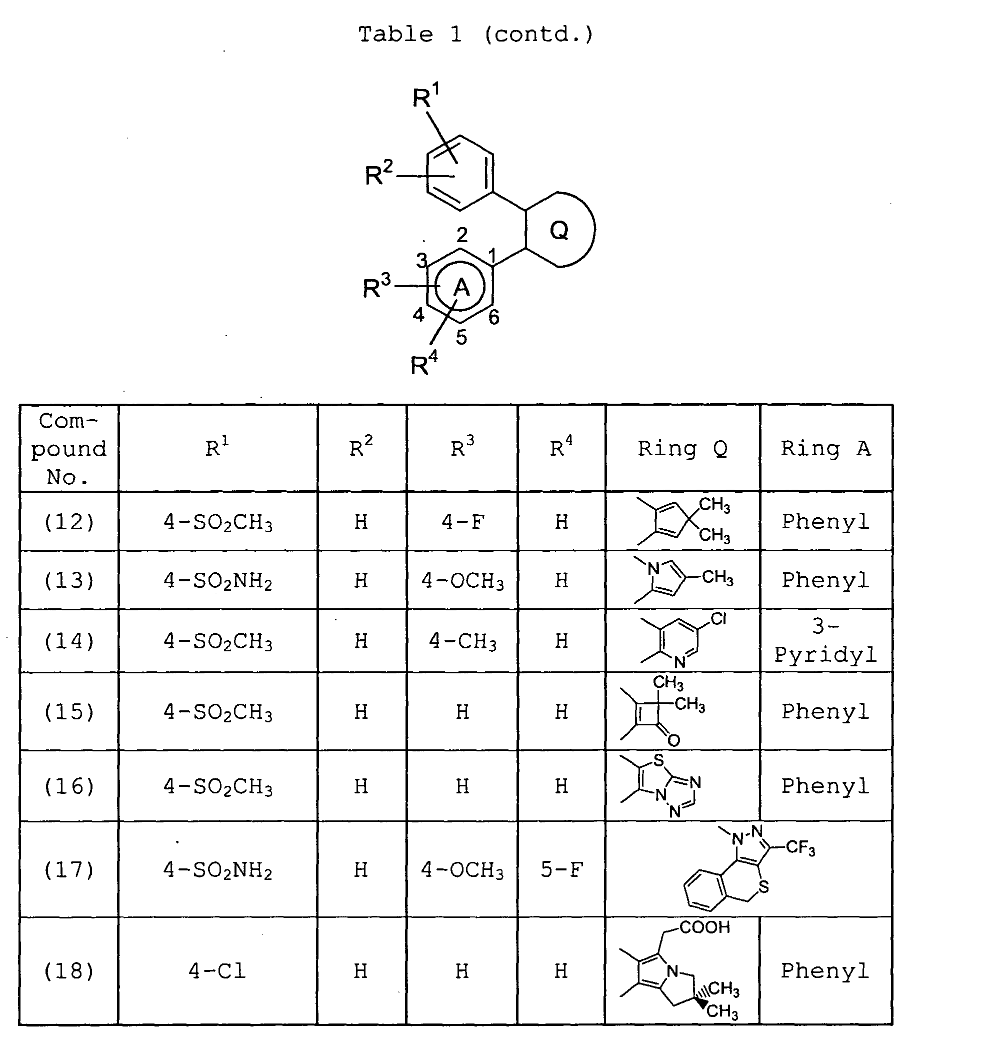

The compounds (1) to (18) can be prepared according to the preparation methods disclosed in the following publications.
(1) International Publication WO95/15318 pamphlet (Japanese PCT Provisional Patent Publication No. 9-506350),
(2) International Publication WO95/00501 pamphlet (Japanese PCT Provisional Patent Publication No. 9-500372),
(3) International Publication WO97/38986 pamphlet (Japanese PCT Provisional Patent Publication No. 2000-509029),
(4) International Publication WO96/25405 pamphlet (Japanese PCT Provisional Patent Publication No. 11-503722),
(5) International Publication WO96/19463 pamphlet (Japanese Provisional Patent Publication No. 9-52882),
(6) International Publication WO00/23426 pamphlet (Japanese PCT Provisional Patent Publication No. 2002-527508),
(7) European Patent 863134 A (Japanese Provisional Patent Publication No. 10-251220),
(8) International Publication WO95/11883 pamphlet (Japanese PCT Provisional Patent Publication No. 9-504288),
(9) International Publication WO95/15316 pamphlet (Japanese PCT Provisional Patent Publication No. 9-506350),
(10) International Publication WO97/29776 pamphlet (Japanese PCT Provisional Patent Publication No. 2000-504723),
(11) International Publication WO99/14205 pamphlet (Japanese PCT Provisional Patent Publication No. 2001-516750),
(12) International Publication WO95/30656 pamphlet,
(13) European Patent 799823 A (Japanese Provisional Patent Publication No. 9-323971),
(14) International Publication WO98/03484 pamphlet (Japanese PCT Provisional Patent Publication No. 11-514008),
(15) International Publication WO97/36863 pamphlet (Japanese PCT Provisional Patent Publication No. 2000-510444),
(16) International Publication WO96/09304 pamphlet,
(17) International Publication WO96/41626 pamphlet (Japanese PCT Provisional Patent Publication No. 11-507670),
(18) Japanese Provisional Patent Publication No. 3-141261.

Also, the compounds of the formulae (I) and (II) can be prepared according to the preparation methods disclosed in the above-mentioned publications. For example, the following compounds (19) to (46) shown in Table 2 were prepared.

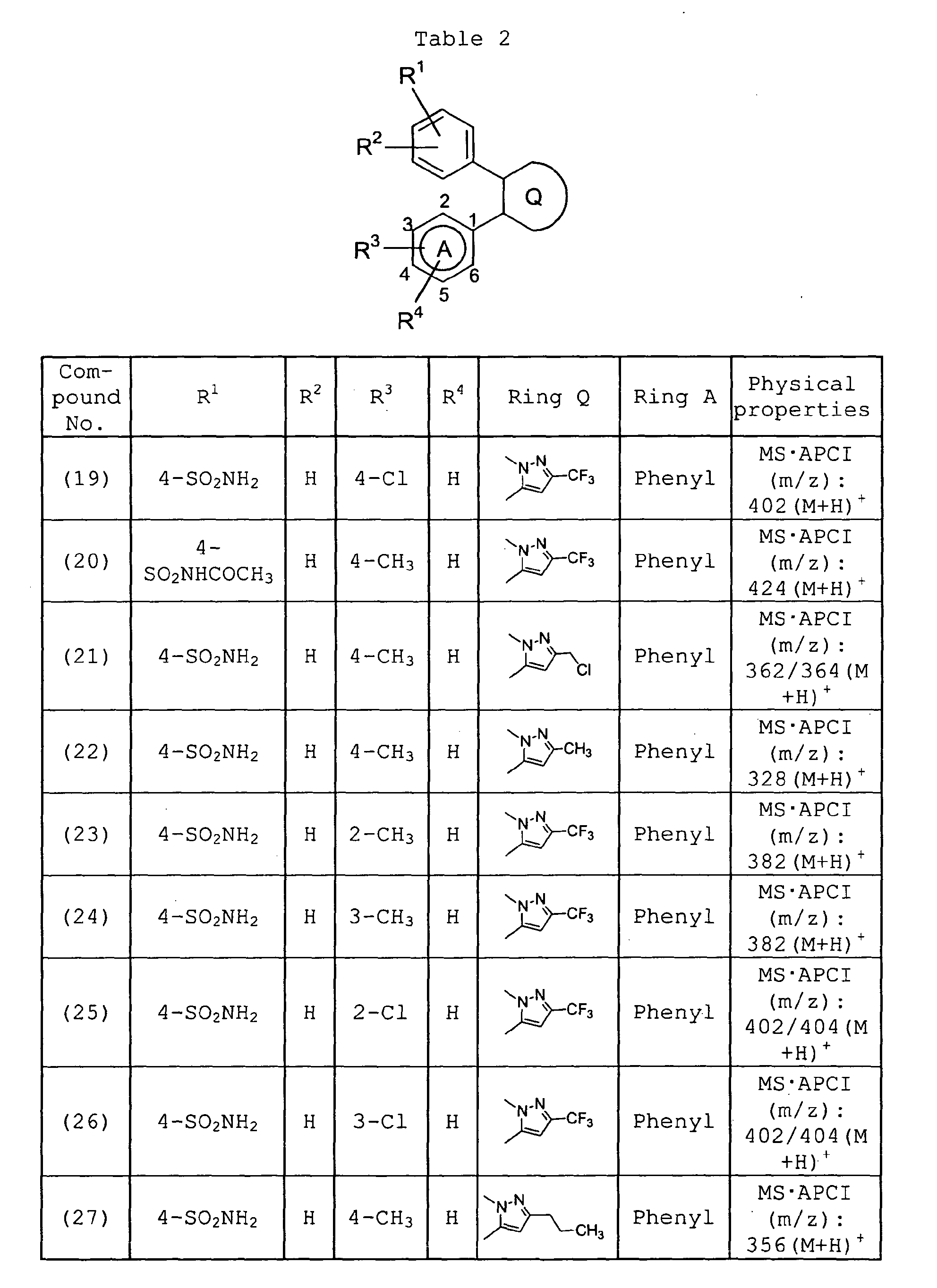

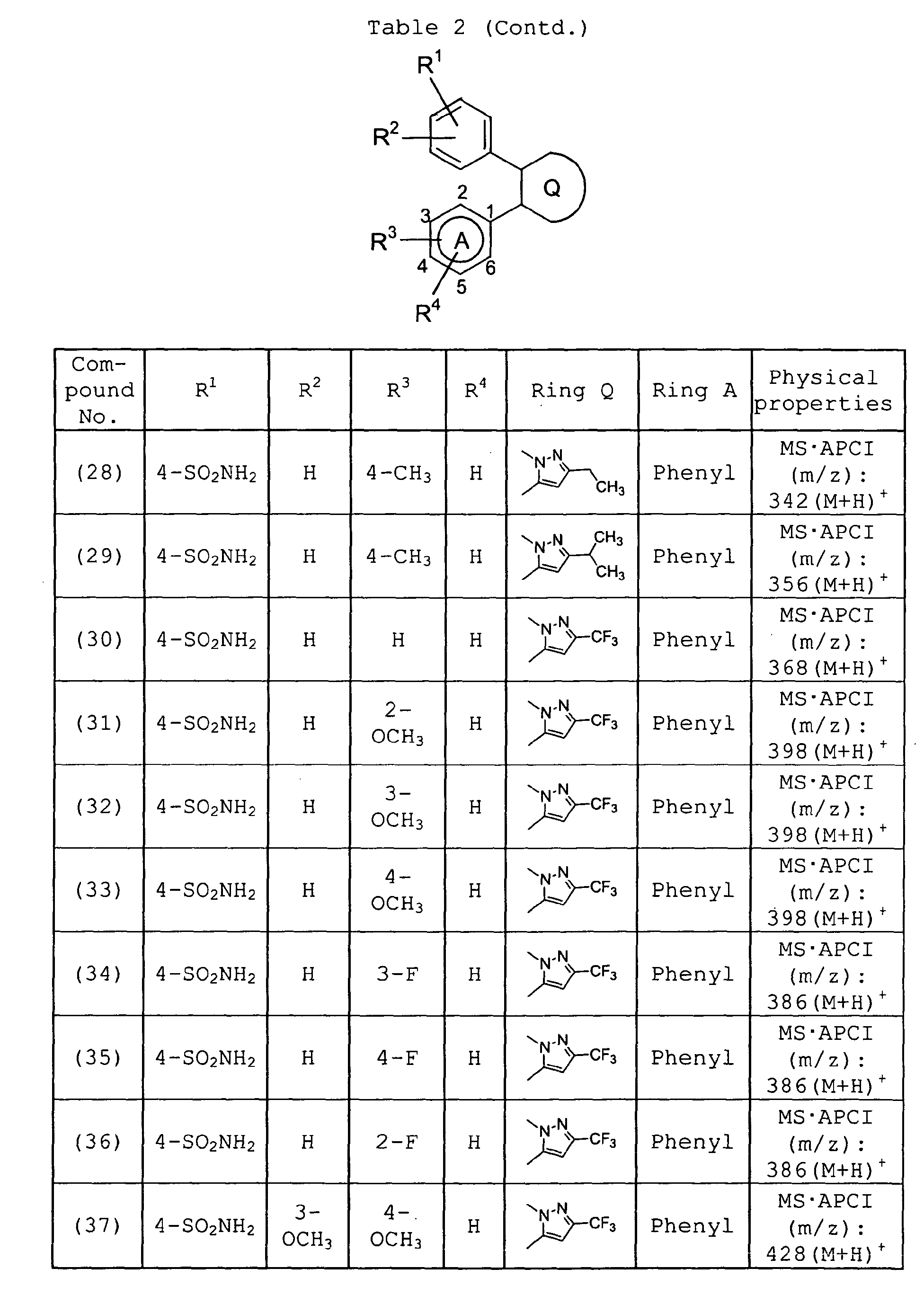

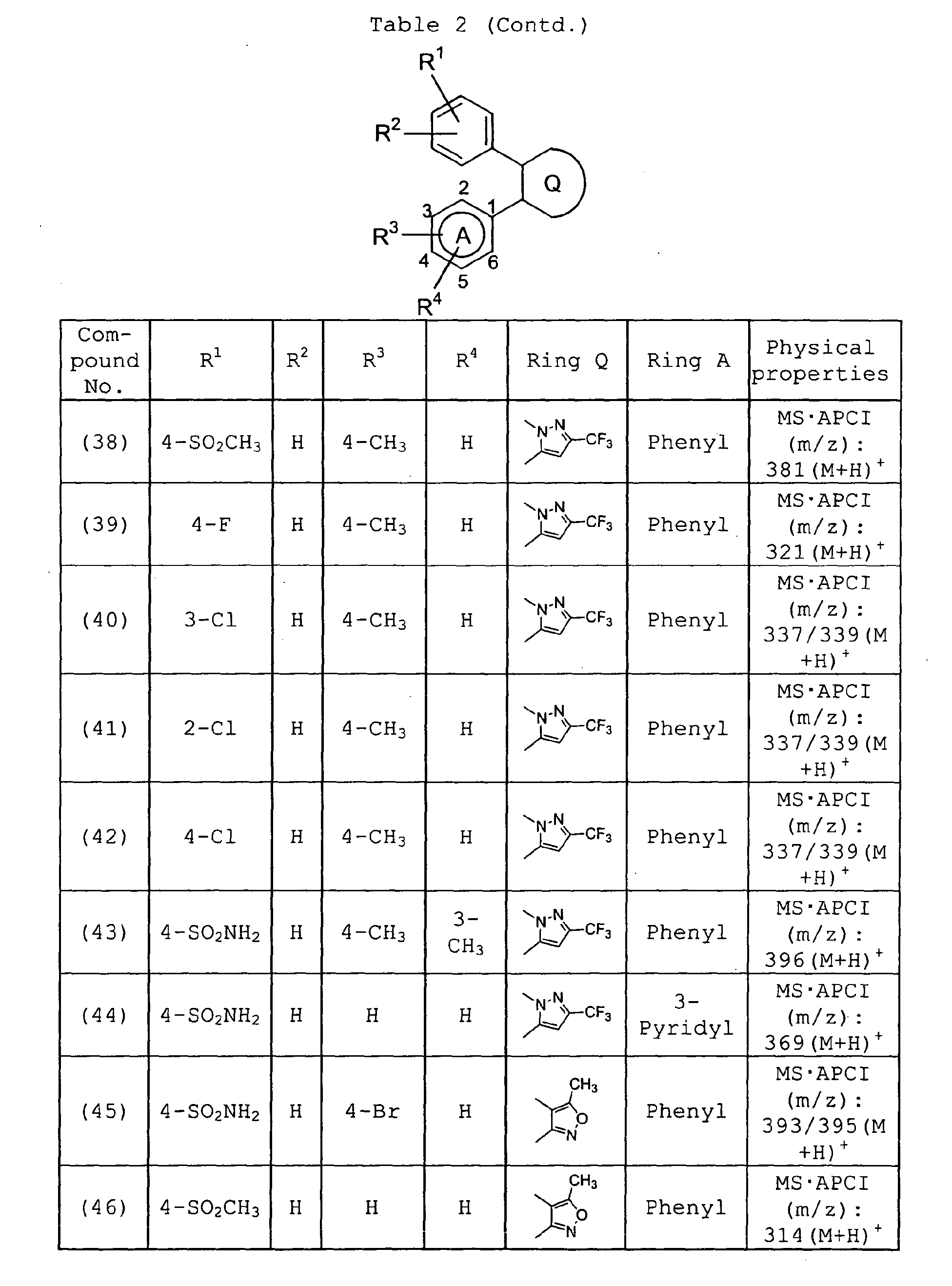

The active ingredient compounds of the present invention or pharmaceutically acceptable salts thereof can be administered orally or parenterally with a pharmaceutically acceptable carrier or diluent and used as common pharmaceutical preparations such as tablets, granules, capsules, powders, injection and inhalants.

As a pharmaceutically acceptable carrier for a preparation of oral administration, there may be mentioned a material commonly used, for example, a binder (such as syrup, Gum Arabic, gelatin, sorbit, tragacanth and polyvinyl pyrrolidone), an excipient (such as lactose, sugar, corn starch, potassium phosphate, sorbit and glycine), a lubricant (such as magnesium stearate, talc, polyethylene glycol and silica), a disintegrator (such as potato starch) and a humectant (such as anhydrous lauryl sodium sulfate).

On the other hand, when the active ingredient compounds of the present invention are administered parenterally, it may be formulated into the form of an injection or a drip infusion by using distilled water for injection, physiological saline, an aqueous glucose solution and the like, or a suppository.

A dose of the active ingredient compounds of the present invention or pharmaceutically acceptable salts thereof may vary depending on an administration method, an age, weight or conditions of a patient, or a kind or degree of disease, and is generally about 0.01 to 50 mg/kg per day, more preferably about 0.1 to 30 mg/kg per day.

The active ingredient compounds of the present invention or pharmaceutically acceptable salts thereof have an excellent large conductance calcium-activated K channel opening activity and hyperpolarizes a membrane electric potential of cells, so that it may be used for the prophylactic, relief and/or treatment of, for example, hypertension, premature birth, irritable bowel syndrome, chronic heart failure, angina, cardiac infarction, cerebral infarction, subarachnoid hemorrhage, cerebral vasospasm, cerebral hypoxia, peripheral blood vessel disorder, anxiety, male-pattern baldness, erectile dysfunction, diabetes, diabetic peripheral nerve disorder, other diabetic complication, sterility, urolithiasis and pain accompanied thereby, pollakiuria, urinary incontinence, nocturnal enuresis, asthma, chronic obstructive pulmonary disease (COPD), cough accompanied by asthma or chronic obstructive pulmonary disease (COPD), cerebral apoplexy, cerebral ischemia, traumatic encephalopathy, and the like.

### BEST MODE OF EMBODIMENT OF THE INVENTION

In the following, the large conductance calcium-activated K channel opener of the present invention is explained in more detail by referring to Experimental examples, but the present invention is not limited by these.

### Experimental example 1

### Relaxation effect on potassium-induced contraction of isolated rabbit urinary bladder

Urinary bladder was isolated from rabbits (weight: 2.0 to 3.5 kg) and immediately immersed in ice-cold Krebs-bicarbonate solution (in mM: 118 NaCl, 4.7 KCl, 2.55 CaCl₂, 1.18 MgSO₄, 1.18 KH₂PO₄, 24.88 NaHCO₃, and 11.1 glucose) to remove peripheral binding tissues. The urinary bladder was cut into longitudinal strips (about 5 mm length, 3 to 4 mm width) after mucosal layer was removed.

Preparations were mounted in organ baths containing Krebs solution maintained at 37°C and gassed with 95% O₂/5% CO₂.

Accordingly, preparations were stretched with an initial tension of 2.0 g, and changes in isometric tension were measured by force-displacement transducer. The preparations were pre-contracted by changing organ-bath solution into high-K⁺ (30 mM) Krebs solution.

After stable tension was obtained, compounds were added into organ baths cumulatively (10⁻⁸ M-10⁻⁴ M). The effects of compounds were expressed as a percentage of the maximum relaxation produced by 0.1 mM papaverine. 50% relaxation concentration (IC₅₀) was calculated and IC₅₀ value (µM) of the compounds of the present invention was shown in the following Table 3.

**Table 3**

| Compound to be tested | IC₅₀ (µM) |
|---|---|
| Compound (1) | 9.24 |
| Compound (3) | 1.53 |
| Compound (23) | 0.11 |

### Experimental example 2

### Inhibitory effect on the rhythmic bladder contractions induced by substance P in anesthetized rats

For the experiments, Sprague-Dawley female rats (9 to 12 weeks old) weighing between 200 to 300 g were used. After urethane anesthetization (subcutaneously administered with a dose of 1.2 g/kg), cannulae were placed in both right and left femoral veins. One intravenous catheter was used for administration of compounds, and the other was for the substance P (0.33 µg/kg/min) infusion. We also cannulated into ureter to pass urine. Polyethylene catheters were inserted into carotid artery for continuous monitoring of arterial blood pressure and heart rate. For continuous infusion, transurethral bladder catheter was inserted into the bladder through the urethra and tied in place by a ligature around the urethral orifice. One end of the catheter was attached to a pressure transducer in order to measure intravesical pressure. The other end of the catheter was used for infusion of saline into the bladder. After stabilization of blood pressure and heart rate and after the bladder was emptied, cystometry was performed by filling the bladder slowly with about 0.6 ml of saline. After about 10 minutes, intravenous infusion of substance P (0.33 µg/kg/min) was started for stabilization of the micturition reflex. Compounds were administered after stable rhythmic bladder contraction was obtained over 15 minutes. All compounds were dissolved or suspended in saline containing 0.5% Tween 80 for intravenous administration (0.1 ml/kg). The rhythmic contraction frequency and the intravesical pressure were observed for 35 minutes after administration of the test compound.

As a result, the compounds of the present invention decreased the frequency of bladder rhythmic contraction without changing the amplitude of contraction. Also, we determined a time (minute) during which the frequency of the rhythmic contraction had been completely inhibited by administering 0.25 mg/kg of the compound. A 100% inhibition time (minute) of the selected compounds of the present invention is shown in the following Table 4.

**Table 4**

| Compound to be tested | Time (min) |
|---|---|
| Compound (1) | 11 |
| Compound (3) | 16.7 |
| Compound (23) | 8 |

Also, it was shown that an inhibitory activity of causing the bladder rhythmic contraction had been weakened by pre-administration of iberiotoxin (0.15 mg/kg, intravenous administration) which is a specific inhibitor for a large conductance calcium-activated K channel opener. Accordingly, it has been shown that the active ingredient of the present invention is effective for a prophylaxis or treatment agent for pollakiuria or urinary incontinence through a large conductance calcium-activated K channel opening activity.

Of the compounds shown by the formulae (I) and (II), other preferred compounds may be mentioned in the following Table 5.

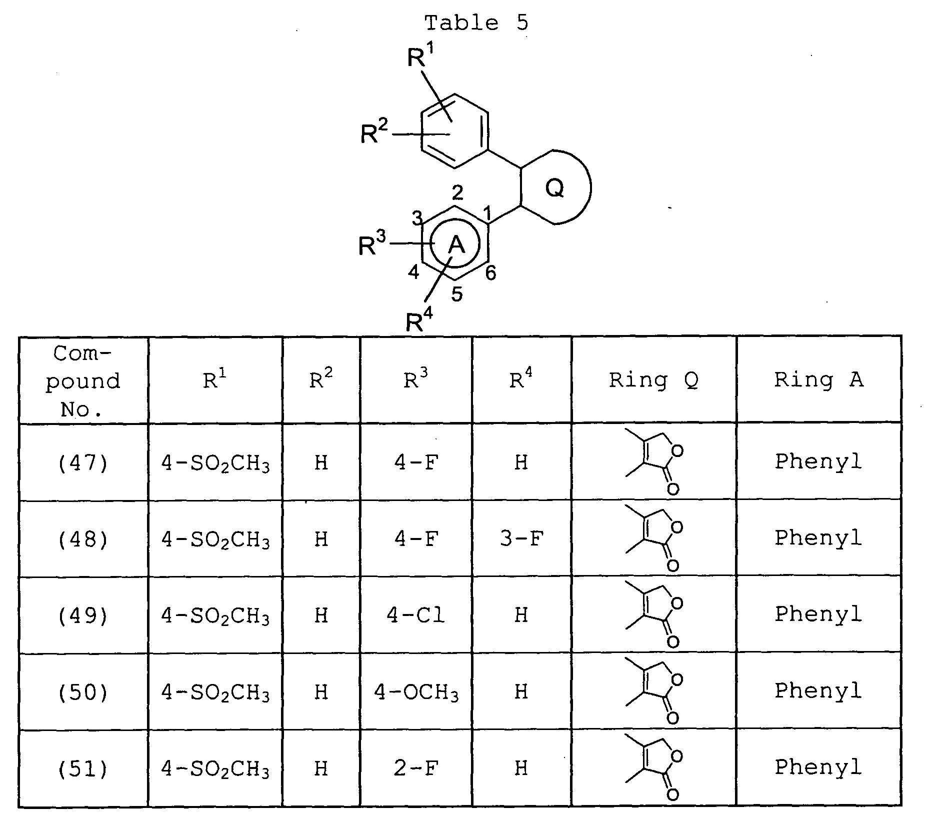

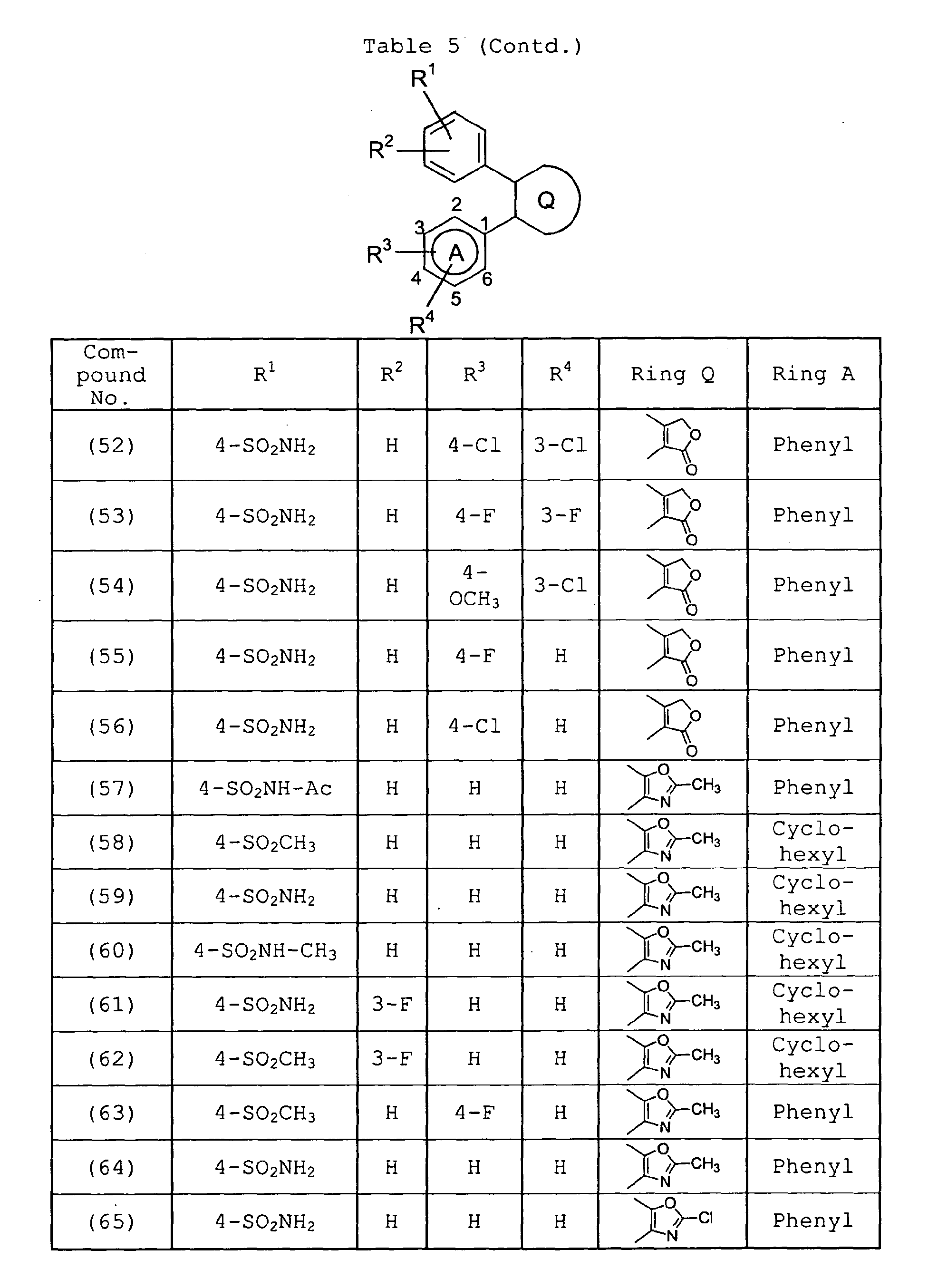

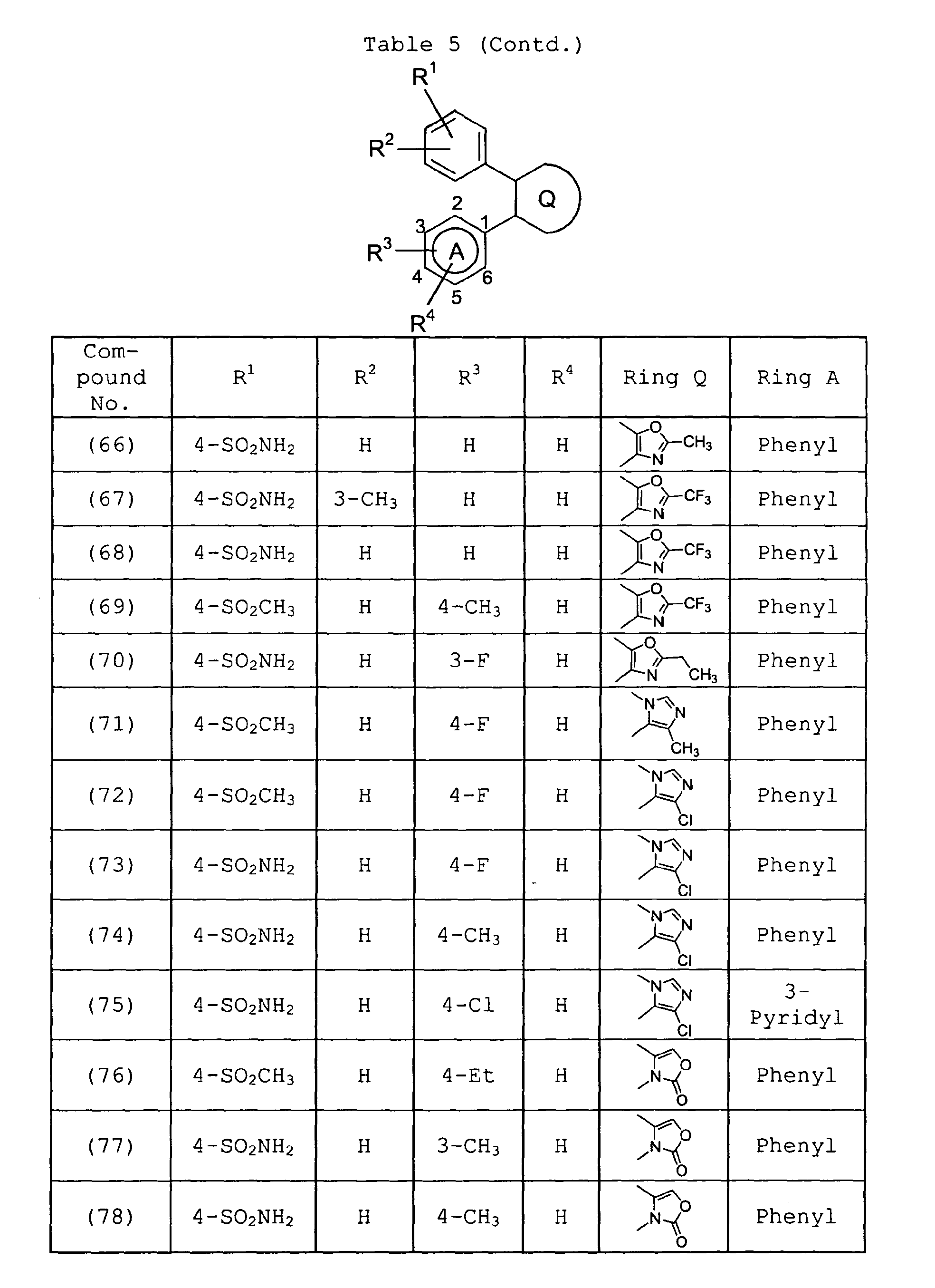

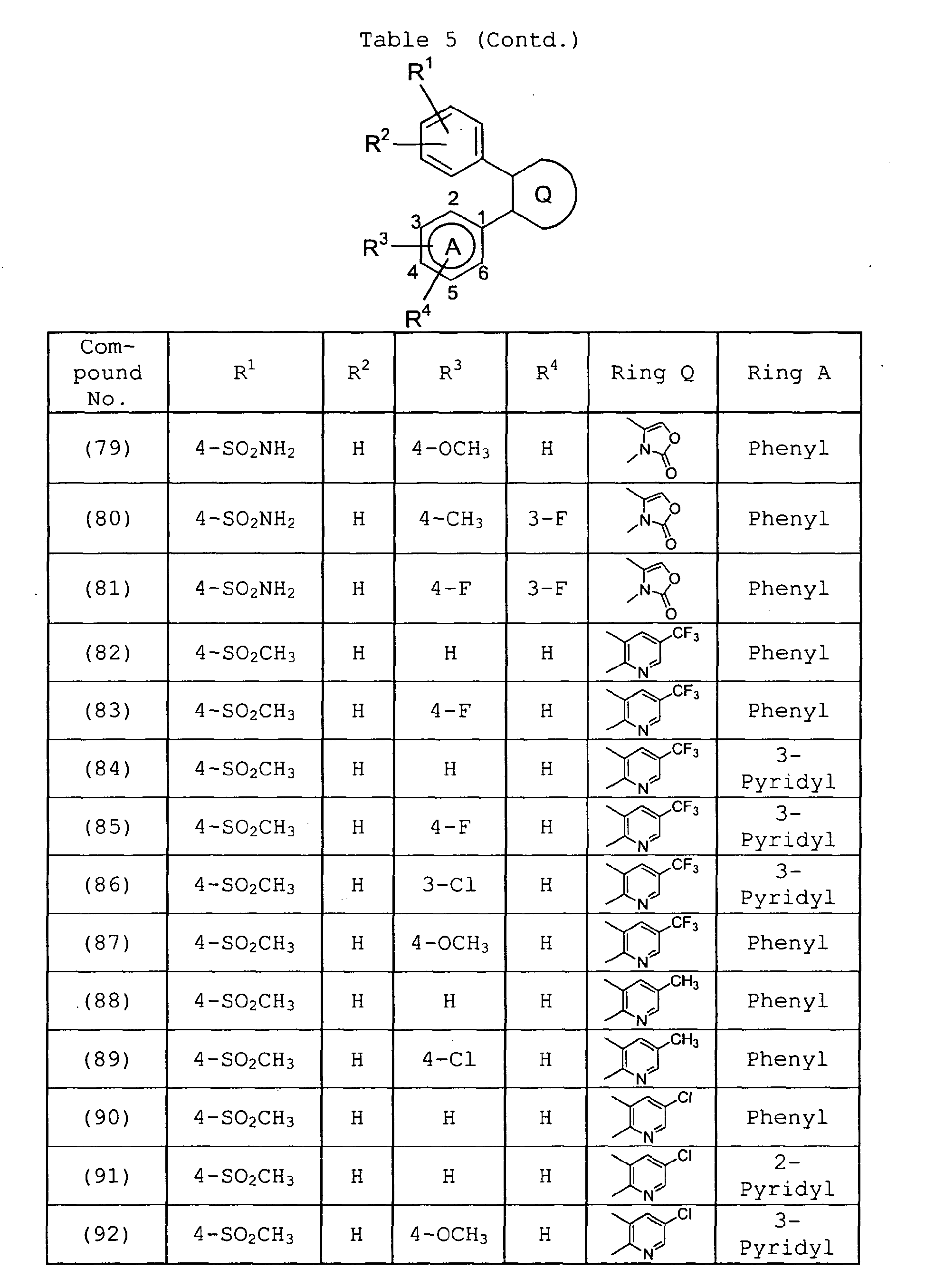

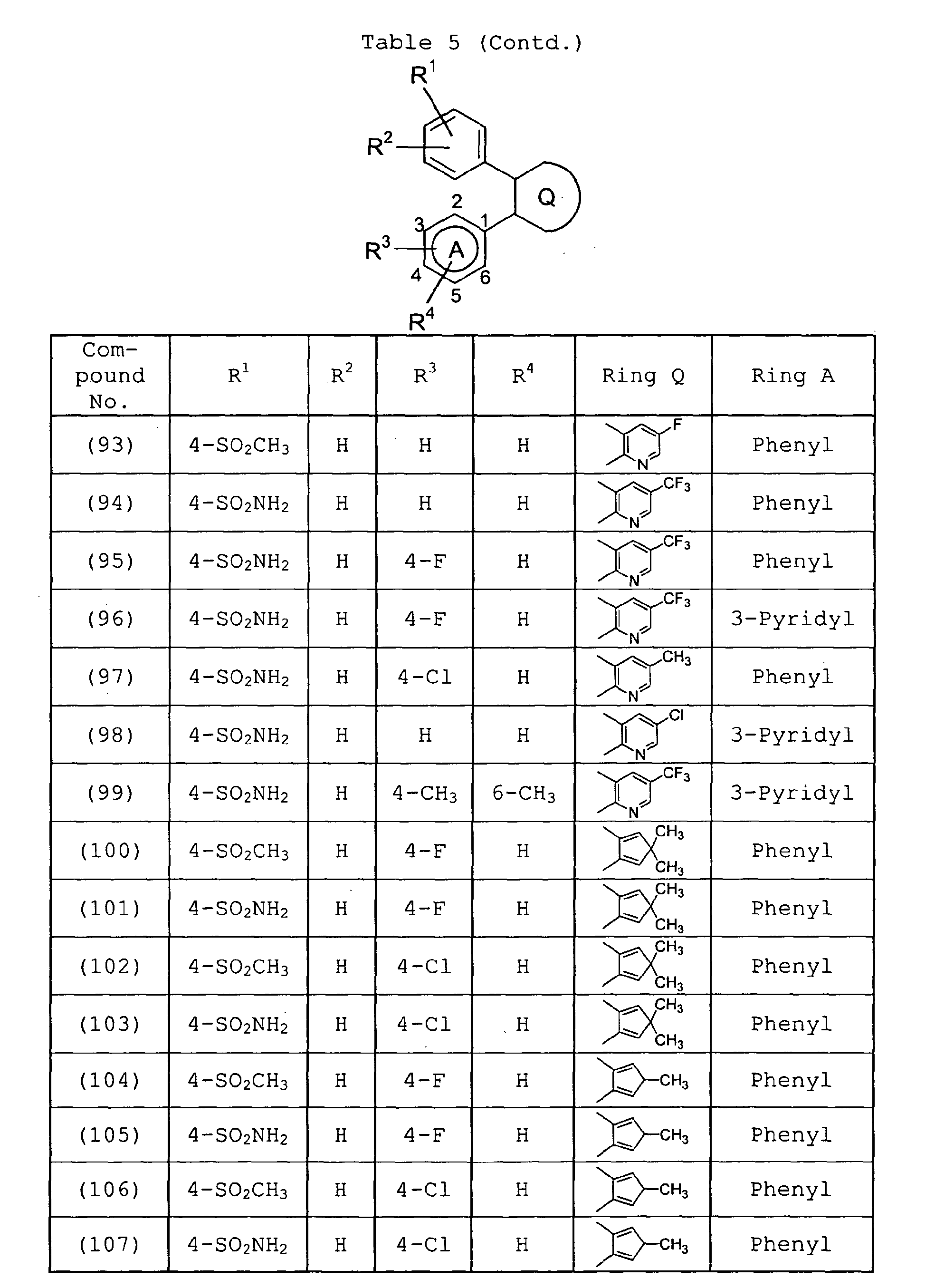

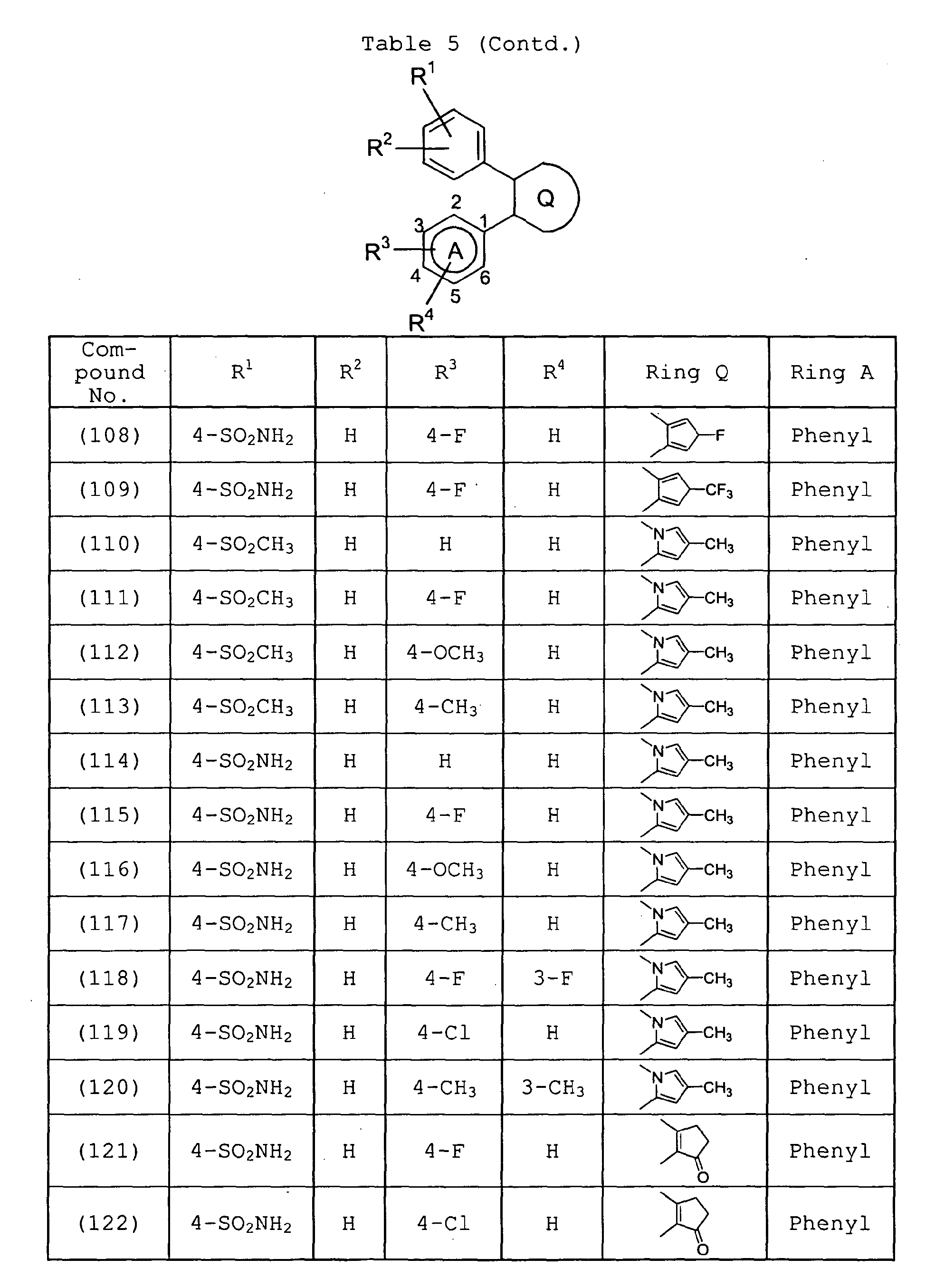

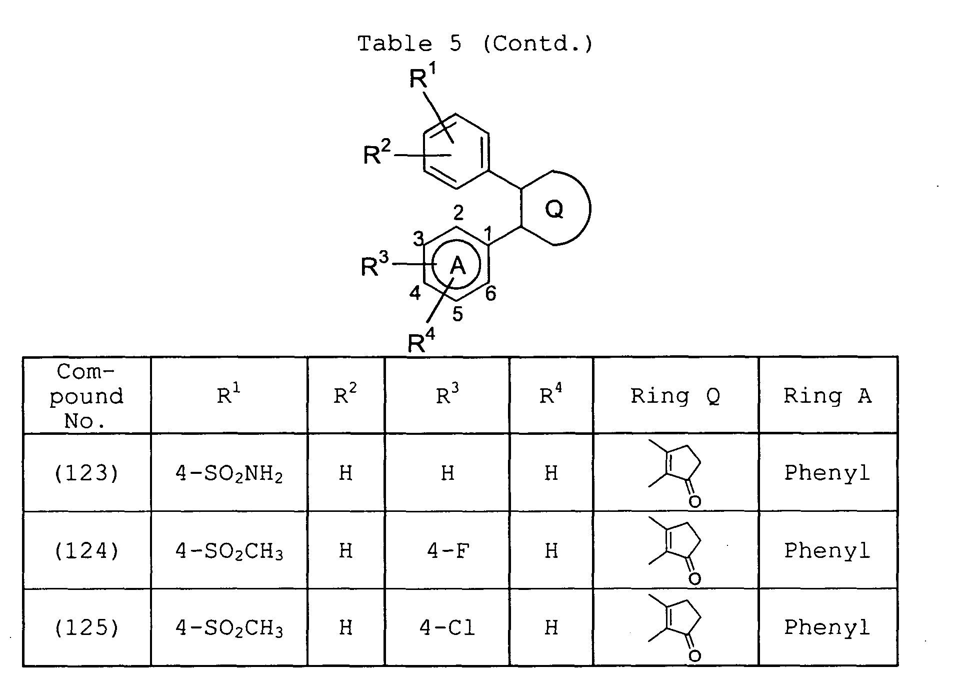

### INDUSTRIAL APPLICABILITY

The active ingredient compounds of the present invention or pharmaceutically acceptable salts thereof have an excellent large conductance calcium-activated K channel opening activity, so that it is used for the prophylactic, relief and/or treatment of hypertension, premature birth, irritable bowel syndrome, chronic heart failure, angina, cardiac infarction, cerebral infarction, subarachnoid hemorrhage, cerebral vasospasm, cerebral hypoxia, peripheral blood vessel disorder, anxiety, male-pattern baldness, erectile dysfunction, diabetes, diabetic peripheral nerve disorder, other diabetic complication, sterility, urolithiasis and pain accompanied thereby, pollakiuria, urinary incontinence, nocturnal enuresis, asthma, chronic obstructive pulmonary disease (COPD), cough accompanied by asthma or chronic obstructive pulmonary disease (COPD), cerebral apoplexy, cerebral ischemia, traumatic encephalopathy, and the like. Also, the active ingredient compounds of the present invention or pharmaceutically acceptable salts thereof have a low toxicity, so that it has high safety as a medicine.

## Claims

1. Use of a compound of formula (I): 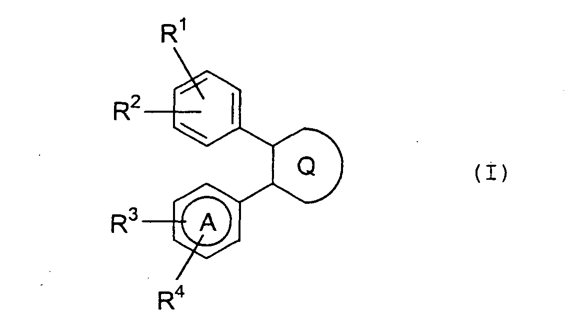 wherein R¹ is a halogen, aminosulfonyl, an alkylsulfonyl or an alkanoylaminosulfonyl; R² is hydrogen or a halogen; R³ and R⁴ may be the same or different from each other and each is hydrogen, a halogen, an alkyl or an alkoxy; Ring A is benzene, pyridine or a cycloalkane, and Ring Q is 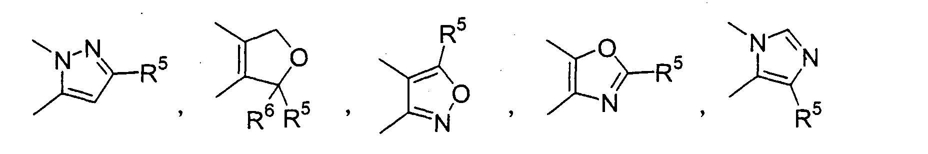 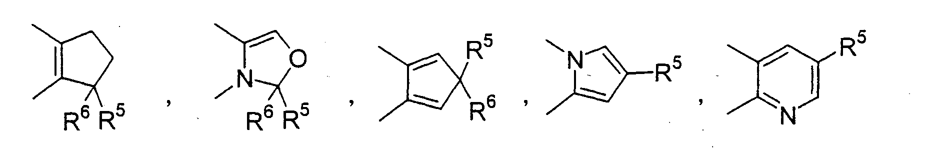 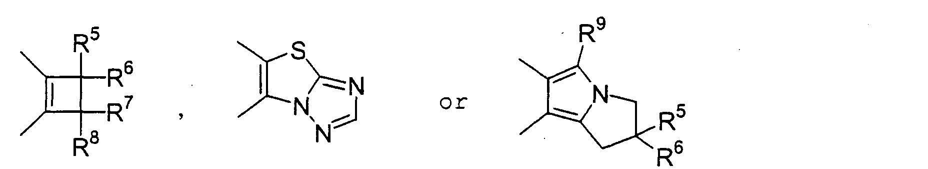 where R⁵ is a halogen, an alkyl or a haloalkyl; R⁶ is hydrogen or an alkyl; or R⁵ and R⁶ may be combined to each other to form oxo; R⁷ and R⁸ are hydrogen or may be combined to each other to form oxo; and R⁹ is a carboxyalkyl,
or Ring Q and Ring A may be combined to each other to form a fused ring of the formula: 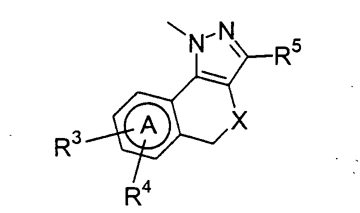 where X is sulfur or oxygen, and R³, R⁴ and R⁵ have the same meanings as defined above,
or a pharmaceutically acceptable salt thereof, for the manufacture of a medicament for the prophylaxis or treatment of a disorder or disease responsive to opening of the large conductance calcium-activated K channel.

2. Use according to claim 1, wherein the compound of formula (I) is a compound of formula (II): 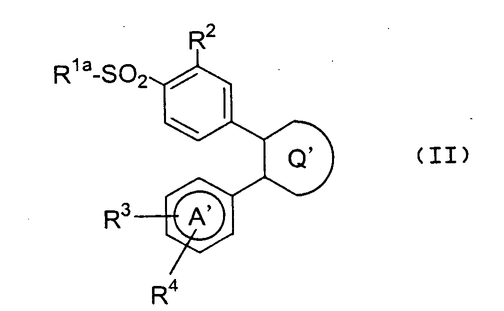 wherein R^{1a} is amino, an alkyl or an alkanoylamino; R² is hydrogen or a halogen; R³ and R⁴ may be the same or different from each other and each is hydrogen, a halogen, an alkyl or an alkoxy; Ring A' is benzene or a cycloalkane, and Ring Q' is 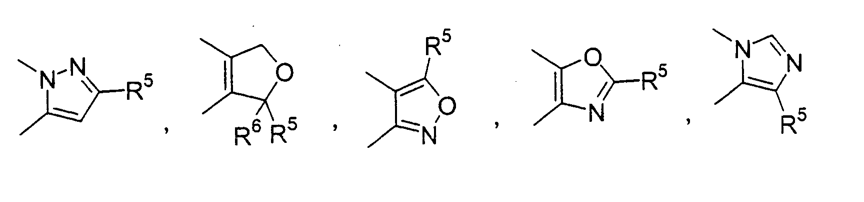 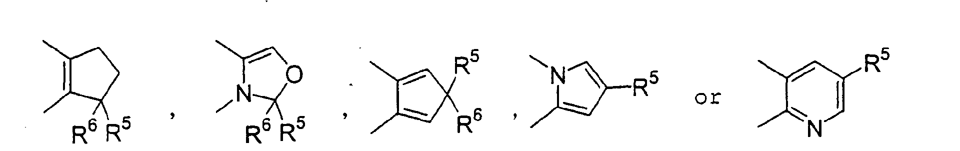 where R⁵ is a halogen, an alkyl or a haloalkyl; R⁶ is hydrogen or an alkyl; or R⁵ and R⁶ may be combined to each other to form oxo.

3. Use according to claim 1, wherein the compound of formula (I) is a compound selected from the group consisting of:
(1) celecoxib,
(2) rofecoxib,
(3) valdecoxib,
(4) parecoxib,
(5) tilmacoxib,
(6) 4-(4-chloro-5-(3-fluoro-4-methoxyphenyl)imidazol-1-yl)benzenesulfonamide,
(7) 2-(3,5-difluorophenyl)-3-(4-methylsulfonylphenyl)-2-cyclopenten-1-one,
(8) 1-fluoro-4-(2-(4-methylsulfonylphenyl)-1-cyclopenten-1-yl)benzene,
(9) 4-(5-(4-chlorophenyl)-3-trifluoromethyl-1H-pyrazol-1-yl)benzenesulfonamide,
(10) 4-(2-methyl-4-phenyloxazol-5-yl)benzenesulfonamide,
(11) 4-(2-oxo-3-phenyl-2,3-dihydroxazol-4-yl)benzene-sulfonamide,
(12) 1-(3,3-dimethyl-5-(4-methylsulfonylphenyl)cyclopenta-1,4-dien-1-yl)-4-fluorobenzene,
(13) 4-(2-(4-methoxyphenyl)-4-methylpyrrol-1-yl)benzene-sulfonamide,
(14) etoricoxib,
(15) 4,4-dimethyl-2-phenyl-3-(4-methylsulfonylphenyl)cyclo-butanone,
(16) 5-(4-methylsulfonylphenyl)-6-phenyl[1,3]thiazole[3,2-b][1,2,4]triazole,
(17) 4-(6-fluoro-7-methoxy-3-trifluoromethylisothio-chromeno[4,3-c]pyrazol-1(5H)-yl)benzenesulfonamide,
(18) licofelone,
(19) 4-[5-(4-chlorophenyl)-3-trifluoromethyl-1H-pyrazol-1-yl]benzenesulfonamide,
(20) N-acetyl-4-[5-(4-methylphenyl)-3-trifluoromethyl-1H-pyrazol-1-yl]benzenesulfonamide,
(21) 4-[5-(4-methylphenyl)-3-chloromethyl-1H-pyrazol-1-yl]-benzenesulfonamide,
(22) 4-[5-(4-methylphenyl)-3-methyl-1H-pyrazol-1-yl]benzenesulfonamide,
(23) 4-[5-(2-methylphenyl)-3-trifluoromethyl-1H-pyrazol-1-yl]benzenesulfonamide,
(24) 4-[5-(3-methylphenyl)-3-trifluoromethyl-1H-pyrazol-1-yl]benzenesulfonamide,
(25) 4-[5-(2-chlorophenyl)-3-trifluoromethyl-1H-pyrazol-1-yl]benzenesulfonamide,
(26) 4-[5-(3-chlorophenyl)-3-trifluoromethyl-1H-pyrazol-1-yl]benzenesulfonamide,
(27) 4-[5-(4-methylphenyl)-3-n-propyl-1H-pyrazol-1-yl]benzenesulfonamide,
(28) 4-[5-(4-methylphenyl)-3-ethyl-1H-pyrazol-1-yl]benzene-sulfonamide,
(29) 4-[5-(4-methylphenyl)-3-isopropyl-1H-pyrazol-1-yl]benzenesulfonamide,
(30) 4-[5-phenyl-3-trifluoromethyl-1H-pyrazol-1-yl]benzene-sulfonamide,
(31) 4-[5-(2-methoxyphenyl)-3-trifluoromethyl-1H-pyrazol-1-yl]benzenesulfonamide,
(32) 4-[5-(3-methoxyphenyl)-3-trifluoromethyl-1H-pyrazol-1-yl]benzenesulfonamide,
(33) 4-[5-(4-methoxyphenyl)-3-trifluoromethyl-1H-pyrazol-1-yl]benzenesulfonamide,
(34) 4-[5-(3-fluorophenyl)-3-trifluoromethyl-1H-pyrazol-1-yl]benzenesulfonamide,
(35) 4-[5-(4-fluorophenyl)-3-trifluoromethyl-1H-pyrazol-1-yl]benzenesulfonamide,
(36) 4-[5-(2-fluorophenyl)-3-trifluoromethyl-1H-pyrazol-1-yl]benzenesulfonamide,
(37) 4-[5-(3,4-dimethoxyphenyl)-3-trifluoromethyl-1H-pyrazol-1-yl]benzenesulfonamide,
(38) 5-(4-methylphenyl)-1-(4-methylsulfonylphenyl)-3-trifluoromethyl-1H-pyrazole,
(39) 5-(4-methylphenyl)-1-(4-fluorophenyl)-3-trifluoromethyl-1H-pyrazole,
(40) 5-(4-methylphenyl)-1-(3-chlorophenyl)-3-trifluoromethyl-1H-pyrazole,
(41) 5-(4-methylphenyl)-1-(2-chlorophenyl)-3-trifluoromethyl-lH-pyrazole,
(42) 5-(4-methylphenyl)-1-(4-chlorophenyl)-3-trifluoromethyl-1H-pyrazole,
(43) 4-[5-(3,4-dimethylphenyl)-3-trifluoromethyl-1H-pyrazol-1-yl]benzenesulfonamide,
(44) 4-[5-(3-pyridyl)-3-trifluoromethyl-1H-pyrazol-1-yl]-benzenesulfonamide,
(45) 4-[5-methyl-3-(4-bromophenyl)isoxazol-4-yl]benzenesulfonamide, and
(46) 5-methyl-3-phenyl-4-(4-methylsulfonylphenyl)isoxazole.

4. Use according to claim 1, wherein the compound of formula (I) is a compound selected from the group consisting of:
(1) celecoxib,
(2) rofecoxib,
(3) valdecoxib,
(10) 4-(2-methyl-4-phenyloxazol-5-yl)benzenesulfonamide,
(21) 4-[5-(4-methylphenyl)-3-chloromethyl-1H-pyrazol-1-yl]benzenesuifonamide,
(22) 4-[5-(4-methylphenyl)-3-methyl-1H-pyrazol-1-yl]benzenesulfonamide,
(23) 4-[5-(2-methylphenyl)-3-trifluoromethyl-1H-pyrazol-1-yl]benzenesulfonamide,
(36) 4-[5-(2-fluorophenyl)-3-trifluoromethyl-1H-pyrazol-1-yl]benzenesulfonamide,
(37) 4-[5-(3,4-dimethoxyphenyl)-3-trifluoromethyl-1H-pyrazol-1-yl]benzenesulfonamide,
(43) 4-[5-(3,4-dimethylphenyl)-3-trifluoromethyl-1H-pyrazol-1-yl]benzenesulfonamide,
(44) 4-[5-(3-pyridyl)-3-trifluoromethyl-1H-pyrazol-1-yl]-benzenesulfonamide, and
(45) 4-[5-methyl-3-(4-bromophenyl)isoxazol-4-yl]benzene-sulfonamide.

5. Use according to any one of claims 1 to 4, wherein the disorder or disease responsive to opening of the large conductance calcium-activated K channel is hypertension, premature birth, irritable bowel syndrome, chronic heart failure, angina, cardiac infarction, cerebral infarction, subarachnoid hemorrhage, cerebral vasospasm, cerebral hypoxia, peripheral blood vessel disorder, anxiety, male-pattern baldness, erectile dysfunction, diabetes, diabetic peripheral nerve disorder, other diabetic complication, sterility, urolithiasis and pain accompanied thereby, pollakiuria, urinary incontinence, nocturnal enuresis, asthma, chronic obstructive pulmonary disease (COPD), cough accompanied by asthma or chronic obstructive pulmonary disease (COPD), cerebral apoplexy, cerebral ischemia or traumatic encephalopathy.

6. Use according to any one of claims 1 to 4, wherein the disorder or disease responsive to opening of the large conductance calcium-activated K channel is pollakiuria or urinary incontinence.
